(19) Europäisches Patentamt
European Patent Office
Office européen des brevets

(11) EP 0 854 176 B1

(12) **FASCICULE DE BREVET EUROPEEN**

(45) Date de publication et mention de la délivrance du brevet:
**28.04.1999 Bulletin 1999/17**

(51) Int Cl.[6]: **C09C 3/00**, C09C 1/36, A61K 7/00

(21) Numéro de dépôt: **97403012.4**

(22) Date de dépôt: **11.12.1997**

(54) **Procédé de préparation d'un composé photochrome, le composé photochrome obtenu et la composition cosmétique le comprenant**

Verfahren zur Herstellung einer photochromen Verbindung, die erhaltene photochrome Verbindung und diese enthaltende kosmetische Zusammensetzung

Process for preparing a photochromic compound, the obtained photochromic compound and the cosmetic composition containing it

(84) Etats contractants désignés:
**DE ES FR GB IT**

(30) Priorité: **16.01.1997 FR 9700413**

(43) Date de publication de la demande:
**22.07.1998 Bulletin 1998/30**

(73) Titulaire: **L'OREAL**
**75008 Paris (FR)**

(72) Inventeur: **Remy, Christophe**
**94400 Vitry/S/Seine (FR)**

(74) Mandataire: **Dodin, Catherine**
**L'OREAL-DPI**
**6 rue Bertrand Sincholle**
**92585 Clichy Cédex (FR)**

(56) Documents cités:
**EP-A- 0 624 553** **US-A- 4 572 618**

- **DATABASE WPI Week 9309 Derwent Publications Ltd., London, GB; AN 93-070851 XP002042416 & JP 05 017 152 A (SUMITOMO SHOJI) , 26 janvier 1993 & JP 05 017 152 A (...)**

EP 0 854 176 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

## Description

La présente invention se rapporte à l'amélioration des propriétés photochromes d'un composé initialement photochrome, et à son application dans le domaine des compositions cosmétiques notamment.

Les compositions cosmétiques, notamment les compositions de maquillage telles que les poudres libres ou compactes, les fonds de teint, les fards à joues ou à paupières, les rouges à lèvres ou les vernis à ongles, sont constituées d'un véhicule approprié et de différents agents de coloration destinés à conférer une certaine couleur auxdites compositions avant et/ou après leur application sur la peau, les muqueuses, les semi-muqueuses et/ou les phanères telles que les ongles ou les cheveux.

Pour créer des couleurs, on utilise aujourd'hui une gamme d'agents de coloration assez limitée et notamment des laques, des pigments minéraux ou des pigments nacrés. Les laques permettent d'obtenir des couleurs vives, mais pour la plupart sont instables à la lumière, à la température ou au pH. Certaines présentent également l'inconvénient de tacher la peau de manière disgracieuse après application, par dégorgement du colorant. Les pigments minéraux, en particulier les oxydes minéraux sont au contraire très stables mais donnent des couleurs plutôt ternes et pâles. Pour obtenir des effets colorés, on peut encore employer des pigments nacrés de couleurs variées, mais jamais intenses, qui permettent d'obtenir des effets irisés mais le plus souvent assez faibles.

Il a alors été proposé d'utiliser des composés photochromes dans les compositions de maquillage ou capillaire, de manière à obtenir des changements agréables et variables dans le 'rendu couleur' des maquillages de la peau et/ou des cheveux.

Les composés photochromes sont des composés qui possèdent la propriété de changer de couleur lorsqu'ils sont irradiés par une source lumineuse, puis de reprendre leur couleur initiale, ou une couleur proche, lorsque l'on arrête l'irradiation. De tels composés trouvent notamment une application particulièrement intéressante dans les compositions cosmétiques, en particulier dans les compositions de maquillage telles que les fonds de teint et les fards à joues ou à paupières. En effet, on a constaté que le 'rendu maquillage' d'une peau maquillée est différent selon que l'on se trouve en éclairage naturel ou en éclairage artificiel. Ainsi, un maquillage réalisé en éclairage artificiel paraîtra plus clair en lumière naturelle. A l'inverse, un maquillage réalisé à l'extérieur paraîtra plus sombre dans un endroit éclairé artificiellement.

Les propriétés photochromes d'un composé peuvent être caractérisées à l'aide de deux paramètres, calculés sur la base de la mesure des coordonnées trichromatiques (L, a, et b), de la manière suivante.

Soit un composé ayant initialement les coordonnées(L0, a0, b0).

Dans un premier temps, on irradie, de façon normalisée, ledit composé par une source lumineuse pendant 30 minutes, puis l'on mesure les nouvelles coordonnées (L30, a30 et b30) qui reflètent le changement de couleur suite à l'irradiation. On peut calculer un premier paramètre ΔE30 qui reflète l'aptitude d'un composé à prendre une couleur différente de celle d'origine :

$$\Delta E30 = [\ (L30 - L0)^2 + (a30 - a0)^2 + (b30 - b0)^2]^{1/2}$$

Dans une seconde étape, on place le composé irradié pendant 30 minutes dans l'obscurité complète pendant 30 minutes, puis l'on mesure les nouvelles coordonnées (L60, a60 et b60).

On peut calculer un second paramètre ΔE60 qui reflète le changement de couleur par rapport à l'état avant irradiation:

$$\Delta E60 = [\ (L60 - L0)^2 + (a60 - a0)^2 + (b60 - b0)^2]^{1/2}$$

La valeur Δ(ΔE), égale à la valeur absolue de la différence entre ΔE60 et ΔE30, reflète la faculté d'un composé à revenir, après irradiation et obscurité, à une couleur proche de celle de l'état initial, c'est-à-dire avant irradiation.

Dans l'art antérieur, il a notamment été proposé d'utiliser en cosmétique des composés photochromes organiques, tels que les composés de la famille des spiropyrannes ou des naphtooxazines.

Ces composés photochromes sont particulièrement intéressants dans la mesure où ils permettent d'obtenir un changement rapide de coloration du support sur lequel ils sont appliqués, lorsque ledit support est exposé aux UV par exemple, avec un retour rapide à la couleur initiale lorsque l'exposition aux UV cesse.

On peut ainsi citer le brevet FR1604929 qui décrit des compositions cosmétiques notamment capillaires sous forme d'aérosol, qui contiennent des composés phototropes tels que des nitrobenzylpyridines, des thiosemicarbazones ou des dérivés de spiropyrannes. Après sprayage de ces compositions sur les cheveux et exposition à la lumière du soleil, on obtient une coloration bleu-violet qui redevient jaune pâle dans l'obscurité.

Il a également été proposé, par exemple par le brevet EP359909, des compositions cosmétiques comprenant des composés photochromes minéraux particulier, choisis parmi les oxydes métalliques, leurs hydrates et leurs complexes. En particulier, ce document mentionne l'utilisation d'oxyde de titane traité de manière à le rendre photochrome, dans des compositions de maquillage telles que des poudres et des fonds de teint.

On connaît par ailleurs, par le document US5176905, un procédé permettant l'obtention d'un oxyde de titane photochrome par mélange d'hydroxyde de fer (FeOOH) et de dioxyde de titane, et calcination à 750-850°C.

On connaît encore, par le document EP624553, un procédé de préparation d'oxyde de titane ayant un photochromisme amélioré, consistant à dissoudre un organotitane et un composé organique comprenant au moins un métal, dans un solvant organique, puis à hydrolyser le mélange, récupérer l'hydrolysat et à le calciner à une température de 550-700°C. On obtient ainsi un oxyde de titane photochrome, présentant une différence de couleur qui peut être quantifiée à l'aide du paramètre ΔE dont la valeur est d'au moins 10. La valeur ΔE est la différence mesurée entre la chromaticité avant irradiation et la chromaticité après irradiation pendant 1 heure, sous UV à 2 $mW/cm^2$.

On connaît également, par le document JP05/017152, un procédé de préparation d'un oxyde de titane photochrome, consistant à mélanger des organotitanes avec au moins un métal choisi parmi le fer, le chrome, le cuivre, le nickel, le vanadium ou le manganèse, puis à fritter ledit mélange en présence de composés du sodium. On peut ainsi obtenir un paramètre ΔE amélioré par rapport à l'art antérieur, et notamment supérieur à 10. Le paramètre ΔE est calculé de la même manière que dans EP624553.

Dans ces deux derniers documents, le paramètre ΔE amélioré est celui qui reflète l'aptitude d'un composé à changer de couleur sous irradiation lumineuse. Il n'est nullement fait mention, dans ce document, d'une quelconque amélioration du paramètre Δ(ΔE) qui reflète l'aptitude dudit composé à revenir à un état proche de l'état initial.

Or, on a constaté que les composés photochromes de l'art antérieur, notamment les composés minéraux, bien qu'ils permettent d'obtenir un changement appréciable de la couleur du maquillage, c'est-à-dire un ΔE relativement important, présentent toutefois l'inconvénient suivant : après l'arrêt de l'irradiation lumineuse, la couleur du maquillage ne revient pas toujours d'une manière acceptable à sa couleur initiale, en particulier elle ne revient pas complètement à une couleur identique à ladite couleur initiale. Le composé photochrome après irradiation et obscurité a une couleur sensiblement différente de sa couleur initiale avant irradiation. On peut ainsi considérer que la 'relaxation du composé photochrome dans le noir' est faible ou peu importante. Ceci se caractérise, pour ces composés, par une valeur de Δ (ΔE) faible, de l'ordre de 3-4.

La présente invention a pour but de proposer un procédé particulier, permettant l'amélioration du photochromisme, et en particulier l'amélioration de la valeur Δ(ΔE), d'un composé initialement photochrome, tout en conservant une valeur ΔE30 satisfaisante, c'est-à-dire au moins égale et souvent améliorée, c'est-à-dire la plus élevée possible.

La présente invention a donc pour objet un procédé de préparation d'un composé photochrome ayant un paramètre Δ(ΔE) supérieur ou égal à 7, dans lequel on traite thermiquement à une température de 400-1000°C un composé initialement photochrome de type oxyde métallique, hydrate d'oxyde métallique ou complexe oxyde et/ou hydrate métallique, en présence d'un composant métallique choisi parmi les oxydes et/ou hydroxydes de lithium, de sodium et/ou de potassium.

L'invention a également pour objet un procédé pour améliorer le paramètre Δ(ΔE) d'un composé initialement photochrome de type oxyde métallique, hydrate d'oxyde métallique ou complexe oxyde et/ou hydrate métallique, consistant à traiter thermiquement à une température de 400-1000°C ledit composé en présence d'un composant métallique choisi parmi les oxydes et/ou hydroxydes de lithium, de sodium et/ou de potassium.

Un autre objet de l'invention est le composé photochrome de type oxyde métallique, hydrate d'oxyde métallique ou complexe oxyde et/ou hydrate métallique, susceptible d'être obtenu par l'un de ces procédés.

Un autre objet de l'invention est la composition cosmétique comprenant ledit composé.

Sans être tenu par la présente explication, le photochromisme d'un oxyde de titane dopé peut être illustré de la manière suivante. L'on considère un oxyde de titane photochrome de forme anatase dopé avec des atomes de fer de valence 3+ et 4+ se substituant aux atomes de titane.

Lors d'une irradiation UV, on peut penser que le cation $Fe^{3+}$, se transformant en $Fe^{4+}$, va céder un électron à une entité X qui va se transformer en entité $X^-$, responsable du changement de couleur dudit composé photochrome. On peut penser que X est une lacune d'oxygène dans le réseau de l'anatase. On peut supposer qu'ensuite, lors d'une deuxième phase, des électrons de la bande de valence de l'oxyde de titane vont se déplacer vers la bande de conduction, générant en conséquence, d'une part des électrons libres, d'autre part des lacunes électroniques dans la bande de valence, aussi appelées 'trou' positif, c'est-à-dire un état vacant d'une bande d'énergie, correspondant à une zone avec une charge négative en moins. On sait par ailleurs que lesdits électrons et lesdites lacunes ont majoritairement tendance à se recombiner. La phase finale, phase de 'relaxation dans le noir', correspondant au retour à la couleur initiale, se fait par transformation de $X^-$ en X, avec libération d'un électron pour le réseau, par exemple dans un défaut de type anionique associé à une lacune d'oxygène, ou vers un $Fe^{4+}$ formé lors de l'irradiation.

Afin d'illustrer le mécanisme permettant l'amélioration des propriétés photochromes d'un composé donné, on peut proposer l'explication suivante. L'on considère un oxyde de titane photochrome de forme anatase dopé avec des atomes de fer de valence 3+ et 4+, qui se trouvent en substitution du titane de valence 4+ dans le réseau cristallin de l'anatase. Afin de conserver l'électroneutralité du composé, une compensation de charges doit se réaliser, probablement par création de lacunes d'oxygène.
Lorsque l'on applique le procédé selon l'invention, c'est-à-dire lorsque l'on traite thermiquement l'oxyde de titane dopé en présence d'ions de petites tailles et faiblement positivement chargés, on peut supposer que :

- pour compenser l'apport de charge positive, une partie des atomes de fer de valence 4+ se transforment en fer de valence 3+, libérant un électron pour X qui se transforme en X-. On obtient ainsi une augmentation de ΔE30.
- pour compenser l'apport de charge positive, le nombre de lacunes d'oxygène et de défauts anioniques associés augmente; or la relaxation dans le noir se fait par libération d'un électron de $X^-$ vers un tel défaut; leur nombre augmentant grâce au procédé de l'invention, le retour à la couleur initiale est amélioré, d'où un paramètre Δ(ΔE) amélioré.
- grâce au procédé selon l'invention, le fer 3+ dans l'anatase a tendance à être préférentiellement proche d'une lacune d'oxygène; les échanges d'électrons entre ledit fer et ladite lacune seront donc facilités, d'où une augmentation du photochromisme (ΔE30 et Δ(ΔE)).

Le procédé selon l'invention consiste donc à traiter thermiquement un composé initialement photochrome de type oxyde métallique, hydrate d'oxyde métallique ou complexe oxyde et/ou hydrate métallique, en présence d'un composant métallique choisi parmi les oxydes et/ou hydroxydes de lithium, de sodium et/ou de potassium.

Parmi les oxydes métalliques, on peut notamment citer les oxydes de titane, de niobium, de silicium, d'aluminium, de zinc, d'hafnium, de thorium, d'étain, de thallium, de zirconium, de béryllium, de cobalt, de calcium, de magnésium.
Les oxydes et hydrates d'oxydes de titane, aluminium, zinc, zirconium, calcium et magnésium sont préférés.
De manière plus préférentielle, on utilisera le dioxyde de titane qui peut être rendu photochrome à l'aide d'un métal choisi parmi le fer, le chrome, le cuivre, le nickel, le manganèse, le cobalt, le molybdène, tel quel ou sous forme de sel tel qu'un sulfate, un chlorate, un nitrate, un acétate.

Le composant métallique est de préférence choisi parmi les oxydes ou hydroxydes de lithium. On peut en particulier citer l'hydroxyde de lithium LiOH, et le peroxyde de sodium $Na_2O_2$.

Le composant métallique peut être présent à raison de 0,01-30% en poids d'ion métallique, de préférence 0,02-20% en poids, par rapport au poids de composé photochrome à traiter.

Le traitement thermique peut être effectué à une température de 400-1000°C, de préférence de 600-900°C, par exemple pendant 10 minutes à 6 heures, de préférence 2-5 heures.

On obtient ainsi un composé photochrome traité thermiquement dont le paramètre Δ(ΔE) est au moins égal à 7, de préférence supérieur ou égal à 10, préférentiellement supérieur ou égal à 12.

Le composé photochrome traité selon le procédé de l'invention peut être incorporé dans une composition cosmétique en une quantité déterminable aisément par l'homme du métier sur la base de ses connaissances générales, et qui peut notamment être comprise entre 0,01 et 30% en poids par rapport au poids total de la composition, de préférence en une quantité de 1 à 15% en poids.

Ladite composition cosmétique peut se présenter sous la forme d'un produit à appliquer sur les muqueuses, les semi-muqueuses et/ou les tissus kératiniques tels que la peau et les phanères (ongles, cils, sourcils, poils et cheveux). Elle contient donc un milieu cosmétiquement acceptable, c'est-à-dire un milieu compatible avec toutes les matières kératiniques telles que la peau, les ongles, les cheveux, les cils et sourcils, les muqueuses et les semi-muqueuses, et toute autre zone cutanée du corps et du visage. Ledit milieu peut comprendre ou se présenter sous la forme de, notamment, une suspension, une dispersion, une solution en milieu solvant ou hydroalcoolique, éventuellement épaissie voire gélifiée; une émulsion huile-dans-eau, eau-dans-huile, ou multiple; un gel ou une mousse; un gel émulsionné; une dispersion de vésicules notamment lipidiques; une lotion biphase ou multiphase; un spray; une poudre libre, compacte ou coulée; une pâte anhydre. L'homme du métier pourra choisir la forme galénique appropriée, ainsi que sa méthode de préparation, sur base de ses connaissances générales, en tenant compte d'une part de la nature des constituants utilisés, notamment de leur solubilité dans le support, et d'autre part de l'application envisagée pour la composition.

Lorsque la composition se présente sous forme aqueuse, notamment sous forme de dispersion, d'émulsion ou de solution aqueuse, elle peut comprendre une phase aqueuse, qui peut comprendre de l'eau, une eau florale telle que l'eau de bleuet et/ou une eau minérale telle que l'eau de Vittel, les eaux du bassin de Vichy, l'eau d'Uriage, l'eau de la Roche Posay, l'eau de la Bourboule, l'eau d'Enghien-les-Bains, l'eau de Saint Gervais-les-Bains, l'eau de Néris-les-Bains, l'eau d'Allevar-les-Bains, l'eau de Digne, l'eau de Lucas, l'eau de Maizières, l'eau de Neyrac-les-Bains, l'eau de Lons-le-Saunier, les Eaux Bonnes, l'eau de Rochefort, l'eau de Saint Christau, l'eau des Fumades et l'eau de Tercis-les-bains. Ladite phase aqueuse peut comprendre de 0 % à 14 % en poids, par rapport au poids total de la phase aqueuse, d'un monoalcool inférieur en $C_2$-$C_6$ et/ou d'un polyol tel que le glycérol, le butylèneglycol, l'isoprène glycol, le propylèneglycol, le polyéthylèneglycol.
Lorsque la composition selon l'invention se présente sous la forme d'une émulsion, elle peut éventuellement comprendre en outre un tensioactif, de préférence en une quantité de 0,01 à 30% en poids par rapport au poids total de la composition.

Parmi les tensioactifs anioniques qui peuvent être utilisés seuls ou en mélange, on peut citer en particulier les sels alcalins, les sels d'ammonium, les sels d'amines ou les sels d'aminoalcools des composés suivants : les alcoylsulfates, alcoyléther sulfates, alcoylamides sulfates et éthers sulfates, alcoylarylpolyéthersulfates, monoglycérides sulfates, alcoylsulfonates, alcoylamides sulfonates, alcoylarylsulfonates, $\alpha$-oléfines sulfonates, paraffines sulfonates, alcoylsulfosuccinates, alcoyléthersulfosuccinates, alcoylamides sulfosuccinates, alcoylsulfosuccinamatés, alcoylsulfoacétates, alcoylpolyglycérol carboxylates, alcoylphosphatés/alcoylétherphosphates, acylsarcosinates, alcoylpolypeptidates, alcoylamidopolypeptidates, acyliséthionates, alcoyllaurates. Le radical alcoyle ou acyle dans tous ces composés désigne généralement une chaîne de 12 à 18 atomes de carbone. On peut aussi citer les savons et les sels d'acides gras tels que les acides oléique, ricinoléique, palmitique, stéarique, les acides d'huile de coprah ou d'huile de coprah hydrogénée et notamment les sels d'amines tels que les stéarates d'amines; les acyl lactylates dont le radical acyle comprend 8-20 atomes de carbone; les acides carboxyliques d'éthers polyglycoliques répondant à la formule : Alk-$(OCH_2\text{-}CH_2)_n$-$OCH_2$-COOH sous forme acide ou salifiée où le substituant Alk correspond à une chaîne linéaire ayant de 12 à 18 atomes de carbone et où n est un nombre entier compris entre 5 et 15.

Parmi les tensioactifs non ioniques qui peuvent être utilisés seuls ou en mélange, on peut citer en particulier : les alcools, les alcoylphénols et acides gras polyéthoxylés, polypropoxylés ou polyglycérolés à chaîne grasse comportant 8 à 18 atomes de carbone; des copolymères d'oxydes d'éthylène et de propylène, des condensats d'oxyde d'éthylène et de propylène sur des alcools gras, des amides gras polyéthoxylés, des amines grasses polyéthoxylées, des éthanolamides, des esters d'acides gras de glycol, des esters d'acides gras du sorbitan oxyéthylénés ou non, des esters d'acides gras du saccharose, des esters d'acides gras de polyéthylèneglycol, des triesters phosphoriques, des esters d'acides gras de dérivés de glucose; les alkylpolyglycosides et les alkylamides des sucres aminés; les produits de condensation d'un $\alpha$-diol, d'un monoalcool, d'un alcoylphénol, d'un amide ou d'un diglycolamide avec le glycidol ou un précurseur de glycidol.

La composition selon l'invention peut également comprendre 0 à 5 % en poids, par rapport au poids total de l'émulsion, d'au moins un co-émulsionnant qui peut être choisi parmi le monostéarate de sorbitan oxyéthyléné, des alcools gras tels que l'alcool stéarylique ou l'alcool cétylique, ou des esters d'acides gras et de polyols tels que le stéarate de glycéryle.

La composition selon l'invention peut encore comprendre un ou plusieurs agents épaississants dans des concentrations préférentielles allant de 0 à 6 % en poids, par rapport au poids total de la composition, choisis parmi:

- les biopolymères polysaccharidiques comme la gomme de xanthane, la gomme de caroube, la gomme de guar, les alginates, les celluloses modifiées telles que l'hydroxyéthylcellulose, la méthylcellulose, l'hydroxypropylcellulose et la carboxyméthylcellulose, les dérivés de l'amidon, les dérivés d'éthers de cellulose possédant des groupes ammonium quaternaires, les polysaccharides cationiques;
- les polymères synthétiques comme les acides polyacryliques tels que les polymères poly(méth)acrylates de glycéryl tels que l'HISPAGEL ou le LUBRAGEL des sociétés HISPANO QUIMICA ou GARDIAN, la polyvinylpyrrolidone, l'alcool polyvinylique, les polymères réticulés d'acrylamide et d'acrylate d'ammonium tels que le PAS 5161 ou BOZEPOL C de HOECHST; les copolymères acrylate/octylacrylamide tels que le Dermacryl de National Starch; les polymères à base de polyacrylamide tels que le SEPIGEL 305 de SEPPIC, les polymères réticulés d'acrylamide et de chlorure de méthacryloyloxyéthyltriméthylammonium tels que le SALCARE SC 92 de ALLIED COLLOIDS,
- le silicate de magnésium et d'aluminium.

Selon l'application envisagée, la composition peut comprendre en outre un polymère filmogène. Ceci est notamment le cas lorsque l'on souhaite préparer une composition de type vernis à ongles, mascara, eye-liner ou composition capillaire de type laque. Les polymères peuvent être dissous ou dispersés dans le milieu cosmétiquement acceptable. En particulier, le polymère peut être présent sous forme de solution dans un solvant organique ou sous forme de dispersion aqueuse de particules de polymère filmogène. Ledit polymère peut être choisi parmi la nitrocellulose, l'acétobutyrate de cellulose, les butyralpolyvinyliques, les résines alkydes, les polyesters, les acryliques, les vinyliques, et/ou les polyuréthannes. On peut en particulier citer les copolymères d'acide (méth)acrylique et d'au moins un monomère ester d'acide (méth)acrylique linéaire, ramifié ou cyclique et/ou d'au moins un monomère amide d'acide (méth)acrylique mono ou di-substitué linéaire, ramifié ou cyclique; les copolymères acide (méth)acrylique/(meth)acrylate de tertio-butyle et/ou (méth)acrylate d'isobutyle/(méth)acrylate d'alkyle en $C_1$-$C_4$; les terpolymères et tétrapolymères acide (meth)acrylique/acrylate d'éthyle/ méthacrylate de méthyle; les tétrapolymères méthacrylate de méthyle/acrylate de butyle ou d'éthyle/acrylate ou méthacrylate d'hydroxyéthyle ou de 2-hydroxypropyle/acide (meth)acrylique; les copolymères d'acide acrylique et de méthacrylate d'alkyle en $C_1$-$C_4$ ; les terpolymères de vinyl pyrrolidone, d'acide acrylique et de méthacrylate d'alkyle en $C_{1\text{-}20}$; les copolymères amphotères; les esters vinyliques d'acide ramifiés; les esters vinyliques d'acide benzoïque; les copolymères d'acide (meth)acrylique et d'au moins un monomère oléfinique; les

copolymères de monoacide vinylique et/ou de monoacide allylique. Parmi les résines, on peut citer les résines du type aryl-sulfonamide formaldéhyde ou aryl-sulfonamide époxy; les résines du type acrylique, styrénique, acrylatestyrénique et vinylique.

La composition peut également comprendre au moins un plastifiant, tel que le phosphate de tricrésyle, le benzoate de benzyle, le citrate de triéthyle, le citrate de tributyle, l'acétylcitrate de triéthyle, l'acétylcitrate de triétyl-2 hexyle, le camphre; les éthers de glycol; l'huile de ricin oxyéthylénée à 40 moles d'oxyde d'éthylène; le propylène glycol; le butyl glycol ; l'éthylène glycol monométhyléther acétate; les éthers de propylène glycol; les éthers esters de propylène glycol et d'éthylène glycol; les esters de diacides tels que les phtalates et adipates de diéthyle, dibutyle et de diisopropyle, les tartrates de diéthyle et dibutyle, les succinates de diéthyle et de dibutyle, les sébacates de diéthyle et de dibutyle, les phosphates de diéthyle, de dibutyle et de diéthyl-2 hexyle, l'acétyl citrate de diéthyle ou de dibutyle; les esters de glycérol. Les plastifiants peuvent être généralement présents à une teneur allant de 1 % à 40 % en poids par rapport au poids total de la composition.

La composition selon l'invention peut également comprendre une phase grasse, notamment constituée de corps gras liquides à 25°C, tels que des huiles d'origine animale, végétale, minérale ou synthétique; de corps gras solides à 25°C tels que des cires d'origine animale, végétale, minérale ou synthétique; de corps gras pâteux; de gommes; de leurs mélanges.

Les compositions selon l'invention peuvent ainsi comprendre des huiles volatiles, qui s'évaporeront au contact de la peau, mais dont la présence, dans la composition cosmétique, est utile car elles facilitent l'étalement de la composition lors de l'application sur la peau. De tels agents d'étalement appelés ici "huiles volatiles", sont généralement des huiles ayant, à 25°C, une tension de vapeur saturante au moins égale à 0,5 millibar (soit 50 Pa). De préférence, on utilise des huiles dont le point éclair est suffisamment élevé pour permettre l'utilisation de ces huiles en formulation, et suffisamment bas pour obtenir l'effet évanescent souhaité. On emploie de préférence des huiles dont le point éclair est de l'ordre de 40-100°C.

On peut ainsi citer les huiles siliconées volatiles, telles que :

- les silicones volatiles cycliques ayant de 3 à 8 atomes de silicium et de préférence de 4 à 6. Il s'agit par exemple de la cyclotétradiméthylsiloxane, de la cyclopentadiméthylsiloxane ou de la cyclohexadiméthylsiloxane,
- les cyclocopolymères du type diméthylsiloxane/méthylalkylsiloxane, tels que la SILICONE FZ 3109 vendue par la société UNION CARBIDE, qui est un cyclocopolymère diméthylsiloxane/méthyloctylsiloxane,
- les silicones volatiles linéaires ayant de 2 à 9 atomes de silicium. Il s'agit par exemple de l'hexaméthyldisiloxane, de l'hexylheptaméthyltrisiloxane ou de l'octylheptaméthyltrisiloxane.

On peut également citer les huiles volatiles hydrocarbonées, telles que les isoparaffines et notamment l'isododécane; et des huiles fluorées comme celle commercialisée sous la dénomination GALDEN® (MONTEFLUOS).

On peut également utiliser des huiles non volatiles, parmi lesquelles on peut citer:

- les polyalkyl($C_1$-$C_{20}$) siloxanes et notamment ceux à groupements terminaux triméthylsilyle, de préférence ceux dont la viscosité est inférieure à 0,06 $m^2/s$ parmi lesquels on peut citer les polydiméthylsiloxanes linéaires et les alkylméthylpolysiloxanes tels que la cétyldiméthicone (nom CTFA),
- les silicones modifiées par des groupements aliphatiques et/ou aromatiques, éventuellement fluorés, ou par des groupements fonctionnels tels que des groupements hydroxyles, thiols et/ou amines.
- les huiles de silicone phénylées, notamment celles de formule :

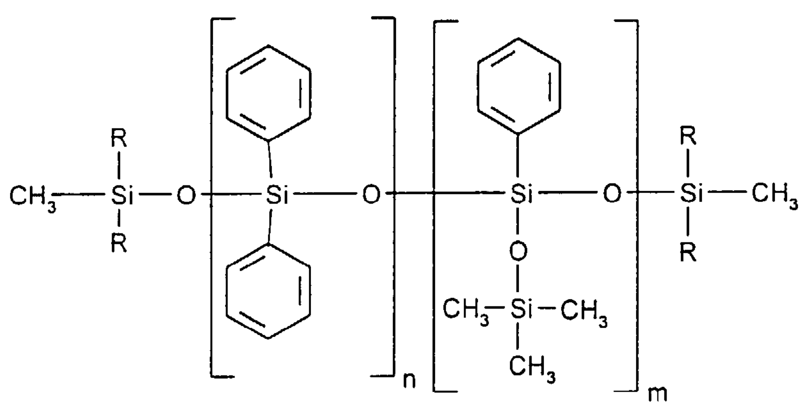

dans laquelle R est un radical alkyle en C1-C30, un radical aryle ou un radical aralkyle, n est un nombre entier compris entre 0 et 100, et m est un nombre entier compris entre 0 et 100, sous réserve que la somme est comprise entre 1 et 100.

- les huiles d'origine animale, végétale ou minérale, et notamment les huiles animales ou végétales formées par des esters d'acide gras et de polyols, en particulier les triglycérides liquides, par exemple les huiles de tournesol, de maïs, de soja, de courge, de pépins de raisin, de sésame, de noisette, d'abricot, d'amandes ou d'avocat; les huiles de poisson, le tricaprocaprylate de glycérol, ou les huiles végétales ou animales de formule $R_1COOR_2$ dans laquelle $R_1$ représente le reste d'un acide gras supérieur comportant de 7 à 19 atomes de carbone et $R_2$ représente une chaîne hydrocarbonée ramifiée contenant de 3 à 20 atomes de carbone, par exemple, l'huile de Purcellin; l'huile de paraffine, de vaseline, le perhydrosqualène, l'huile de germes de blé, de calophyllum, de sésame, de macadamia, de pépins de raisin, de colza, de coprah, d'arachide, de palme, de ricin, de jojoba, d'olive ou de germes de céréales; des esters d'acides gras; des alcools; des acétylglycérides; des octanoates, décanoates ou ricinoléates d'alcools ou de polyalcools; des triglycérides d'acides gras; des glycérides;
- les huiles fluorées et perfluorées.

La composition selon l'invention peut comprendre en outre d'autres corps gras, qui peuvent être choisis par l'homme du métier sur base de ses connaissances générales, de manière à conférer à la composition finale les propriétés souhaitées, par exemple en consistance et/ou en texture. Ces corps gras additionnels peuvent être des cires, des gommes et/ou des corps gras pâteux d'origine animale, végétale, minérale ou synthétique, ainsi que leurs mélanges.
On peut notamment citer:

- les gommes de silicones,
- les cires d'origine animale, végétale, minérale ou synthétique telles que les cires microcristallines, la paraffine, le pétrolatum, la vaseline, l'ozokérite, la cire de montan; la cire d'abeilles, la lanoline et ses dérivés; les cires de Candellila, d'Ouricury, de Carnauba, du Japon, le beurre de cacao, les cires de fibres de lièges ou de canne à sucre; les huiles hydrogénées concrètes à 25°C, les ozokérites, les esters gras et les glycérides concrets à 25°C; les cires de polyéthylène et les cires obtenues par synthèse de Fischer-Tropsch; des huiles hydrogénées concrètes à 25°C; des lanolines; des esters gras concrets à 25°C; les cires de silicone; les cires fluorées.

La composition selon l'invention peut également comprendre un ou plusieurs solvants organiques cosmétiquement acceptables (tolérance, toxicologie et toucher acceptables). Ces solvants organiques peuvent représenter de 0 % à 98 % du poids total de la composition et peuvent être choisis dans le groupe constitué par les solvants organiques hydrophiles, les solvants organiques lipophiles, les solvants amphiphiles ou leurs mélanges.
Parmi les solvants organiques hydrophiles, on peut citer par exemple des monoalcools inférieurs linéaires ou ramifiés ayant de 1 à 8 atomes de carbone comme l'éthanol, le propanol, le butanol, l'isopropanol, l'isobutanol ; des polyéthylène glycols ayant de 6 à 80 oxydes d'éthylène ; des polyols tels que le propylène glycol, l'isoprène glycol, le butylène glycol, le glycérol, le sorbitol ; les mono- ou di-alkyle d'isosorbide dont les groupements alkyle ont de 1 à 5 atomes de carbone ; les éthers de glycol comme le diéthylène glycol mono-méthyl ou monoéthyl éther et les éthers de propylène glycol comme le dipropylène glycol méthyl éther. Comme solvants organiques amphiphiles, on peut citer des polyols tels des dérivés de polypropylène glycol (PPG) tels que les esters de polypropylène glycol et d'acide gras, de PPG et d'alcool gras comme le PPG-23 oléyl éther et le PPG-36 oléate. Comme solvants organiques lipophiles, on peut citer par exemple les esters gras tels que l'adipate de diisopropyle, l'adipate de dioctyle, les benzoates d'alkyle, le myristate d'isopropyle, le palmitate d'isopropyle, le stéarate de butyle, le laurate d'hexyle, l'isononanoate d'isononyle, le palmitate de 2-éthyl hexyle, le laurate de 2-hexyl-décyle, le palmitate de 2-octyl-décyle, le myristate de 2-octyldodécyle, le succinate de di-(éthyl-2-hexyle), le malate de diisostéaryle, le lactate de 2-octyl-dodécyle, le triisostéarate de glycérine, le triisostéarate de diglycérine.
La composition peut comprendre en outre une phase particulaire, qui peut comprendre des pigments et/ou des nacres et/ou des charges habituellement utilisés dans les compositions cosmétiques. Par pigments, il faut comprendre des particules blanches ou colorées, minérales ou organiques, destinées à colorer et/ou opacifier la composition. Par charges, il faut comprendre des particules incolores ou blanches, minérales ou de synthèse, lamellaires ou non lamellaires, destinées à donner du corps ou de la rigidité à la composition, et/ou de la douceur, de la matité et de l'uniformité au maquillage. Par nacres, il faut comprendre des particules irisées qui réfléchissent la lumière.
Les pigments peuvent être présents dans la composition à raison de 0 à 15% en poids de la composition finale, et de préférence à raison de 8 à 10% en poids. Ils peuvent être blancs ou colorés, minéraux et/ou organiques, de taille usuelle ou nanométrique. On peut citer les dioxydes de titane, de zirconium ou de cérium, ainsi que les oxydes de zinc, de fer ou de chrome, le bleu ferrique, l'hydrate de chrome, le noir de carbone, les outremers (polysulfures d'aluminosilicates), le pyrophosphate de manganèse et certaines poudres métalliques telles que celles d'argent ou d'aluminium, le noir de carbone. On peut encore citer les laques couramment employées pour conférer aux lèvres et à la peau un effet de maquillage, qui sont des sels de calcium, de baryum, d'aluminium ou de zirconium, de colorants acides tels que les colorants halogéno-acides, azoïques, anthraquinoniques, etc.
Les nacres peuvent être présentes dans la composition à raison de 0 à 20% en poids, de préférence à un taux de l'ordre de 8 à 15% en poids. Parmi les nacres envisageables, on peut citer la nacre naturelle, le mica recouvert d'oxyde de titane, d'oxyde de fer, de pigment naturel ou d'oxychlorure de bismuth ainsi que le mica titane coloré.
Les charges, qui peuvent être présentes à raison de 0 à 30% en poids, de préférence 5 à 15%, dans la composition, peuvent être minérales ou de synthèse, lamellaires ou non lamellaires. On peut citer le talc, le mica, la silice, le kaolin, les poudres de Nylon et de polyéthylène, le Téflon, l'amidon, le nitrure de bore, les microsphères de polymère telles que l'Expancel (Nobel Industrie), le polytrap (Dow Corning) et les microbilles de résine de silicone (Tospearls de Toshiba, par exemple), le carbonate de calcium précipité, le carbonate ou l'hydrocarbonate de magnésium, des savons métalliques dérivés d'acides organiques carboxyliques ayant de 8 à 22 atomes de carbone.
Selon le type de formulation, la phase pulvérulente peut représenter de 0,01 à 99% en poids de la composition.
La composition peut en outre comprendre un colorant, notamment un colorant organique naturel tel que le carmin de cochenille, et/ou un colorant de synthèse tel que les colorants halogéno-acides, azoïques, anthraquinoniques. On peut également citer des colorants minéraux tels que le sulfate de cuivre.

La composition peut comprendre en outre tout additif usuellement utilisé dans le domaine cosmétique, tel que des antioxydants, des parfums, des huiles essentielles, des conservateurs, des actifs cosmétiques lipophiles ou hydrophiles, des hydratants, des vitamines, des acides gras essentiels, des sphingolipides, des agents autobronzants tels que la DHA, des filtres solaires, des agents antimousses, des agents séquestrants, des antioxydants.
Bien entendu l'homme du métier veillera à choisir les éventuels composés complémentaires, et/ou leur quantité, de manière telle que les propriétés avantageuses de la composition selon l'invention ne soient pas, ou substantiellement pas, altérées par l'adjonction envisagée.

Les compositions cosmétiques peuvent se présenter sous la forme d'un produit de soin et/ou de maquillage de la peau, un produit solaire ou autobronzant, voire un produit capillaire. En particulier, elles trouvent une application particulière dans le domaine des rouges à lèvres, des fonds de teint, des fards à joues ou à paupières, des poudres libres ou compactes, des crèmes teintées, des eye-liners, des mascaras et des vernis à ongles aqueux ou solvant.

L'invention est illustrée plus en détail dans les exemples suivants.

Exemple 1

Le composé photochrome pris comme exemple est l'oxyde de titane dopé au fer vendu par C.C.I.C. par l'intermédiaire d'IKEDA, sous la dénomination 'PHOTOGENICA 1'.
Ses caractéristiques initiales sont les suivantes:

- après 30 minutes d'irradiation avec une lampe émettant à 365 nm : ΔE30 = 12,0
- après 30 minutes d'obscurité : Δ(ΔE) = 4,2

*a/ traitement thermique en présence de LiOH*

On prépare un mélange comprenant l'oxyde de titane dopé au fer et 0,9% en poids de LiOH, correspondant à 0,25% en masse de Li+.
On mixe mécaniquement le mélange au mortier, puis on traite thermiquement pendant 4 heures, dans un four tubulaire, à une température d'environ 800°C.
On obtient les résultats donnés dans le tableau ci-après.

*b/ traitement thermique en présence de Na2O2*

On prépare un mélange comprenant l'oxyde de titane dopé au fer et 30% en poids de $Na_2O_2$, correspondant à 18% en masse de Na+.
On mixe mécaniquement le mélange au mortier, puis on traite thermiquement pendant 4 heures, dans un four tubulaire, à une température d'environ 800°C.
On obtient les résultats donnés dans le tableau ci-après.

| | **ΔE30** | **Δ(ΔE)** |
|---|---|---|
| Oxyde de titane dopé au fer | 12,0 | 4,2 |

(suite)

| | ΔE30 | Δ(ΔE) |
|---|---|---|
| après traitement avec LiOH | 17,0 | 12,0 |
| après traitement avec $Na_2O_2$ | 12,3 | 7,6 |

Le procédé selon l'invention permet donc bien d'améliorer la valeur de Δ(ΔE) tout en conservant une valeur de ΔE30 acceptable, voire améliorée.

<u>Exemple 2</u>

On ajoute à un oxyde de titane pur, de type anatase, dispersé dans 50 ml d'eau :

- 0,41% de fer sous forme de $FeCl_3$ (% calculé en équivalent massique de $Fe_2O_3$ par rapport à l'oxyde de titane), et éventuellement
- 0,05% de lithium, sous forme de LiOH (en masse de Li+ par rapport à l'oxyde de titane).

Le mélange est évaporé à température ambiante sous agitation, de façon à assurer une distribution homogène des ions lithium et fer, puis on traite thermiquement la poudre obtenue pendant 4 heures, dans un four tubulaire, à une température d'environ 800°C.
On obtient les résultats donnés dans le tableau ci-après.

| | ΔE30 | Δ(ΔE) |
|---|---|---|
| avec 0,41% de fer | 10 | 5 |
| avec 0,41% de fer + 0,05% de lithium | 22,6 | 17,2 |

On constate donc que le traitement selon le procédé de l'invention permet l'obtention de propriétés photochromes supérieures à celles obtenues selon l'art antérieur.

<u>Exemple 3</u>

On prépare une poudre compactée ayant la composition suivante :

- talc 30 g
- mica 20 g
- BiOCl 10 g
- poudre de Nylon 16 g
- stéarate de zinc 5 g
- oxyde de fer 2 g
- oxyde de titane selon l'exemple 1 a) 10 g
- liant gras 7 g

On obtient une composition ayant de bonnes propriétés cosmétiques.

**Revendications**

1. Procédé de préparation d'un composé photochrome ayant un paramètre Δ(ΔE) supérieur ou égal à 7, dans lequel on traite thermiquement à une température de 400-1000°C un composé initialement photochrome de type oxyde métallique, hydrate d'oxyde métallique ou complexe oxyde et/ou hydrate métallique, en présence d'un composant métallique choisi parmi les oxydes et/ou hydroxydes de lithium, de sodium et/ou de potassium.

2. Procédé pour améliorer le paramètre Δ(ΔE) d'un composé initialement photochrome de type oxyde métallique, hydrate d'oxyde métallique ou complexe oxyde et/ou hydrate métallique, consistant à traiter thermiquement à une température de 400-1000°C ledit composé en présence d'un composant métallique choisi parmi les oxydes et/ou

hydroxydes de lithium, de sodium et/ou de potassium.

**3.** Procédé selon l'une des revendications précédentes, dans lequel le composé photochrome est choisi parmi les oxydes ou hydrates d'oxydes de titane, de niobium, de silicium, d'aluminium, de zinc, d'hafnium, de thorium, d'étain, de thallium, de zirconium, de béryllium, de cobalt, de calcium, de magnésium.

**4.** Procédé selon l'une des revendications précédentes, dans lequel le composé photochrome est un dioxyde de titane rendu photochrome à l'aide d'un métal choisi parmi le fer, le chrome, le cuivre, le nickel, le manganèse, le cobalt, le molybdène, tel quel ou sous forme de sel tel qu'un sulfate, un chlorate, un nitrate, un acétate.

**5.** Procédé selon l'une des revendications précédentes, dans lequel le composant métallique est choisi parmi les oxydes ou hydroxydes de lithium.

**6.** Procédé selon l'une des revendications précédentes, dans lequel le composant métallique est présent à raison de 0,01-30% en poids d'ion métallique, de préférence 0,02-20%, par rapport au poids de composé photochrome à traiter.

**7.** Procédé selon l'une des revendications précédentes, dans lequel le traitement thermique est effectué à une température de 600-900°C.

**8.** Procédé selon l'une des revendications précédentes, dans lequel le traitement thermique est effectué pendant 10 minutes à 6 heures, de préférence 2-5 heures.

**9.** Composé photochrome de type oxyde métallique, hydrate d'oxyde métallique ou complexe oxyde et/ou hydrate métallique, susceptible d'être obtenu par le procédé selon l'une des revendications 1 à 8.

**10.** Composé selon la revendication 9, ayant un paramètre $\Delta(\Delta E)$ supérieur ou égal à 10, de préférence supérieur ou égal à 12.

**11.** Composition cosmétique comprenant le composé selon l'une des revendications 9 à 10.

**12.** Composition selon la revendication 11, comprenant 0,01 et 30% en poids, de préférence 1 à 15% en poids, de composé selon l'une des revendications 9 à 10.

**13.** Composition selon l'une des revendications 11 à 12, se présentant sous la forme d'une suspension, une dispersion, une solution en milieu solvant ou hydroalcoolique, éventuellement épaissie voire gélifiée; une émulsion huile-dans-eau, eau-dans-huile, ou multiple; un gel ou une mousse; un gel émulsionné; une dispersion de vésicules notamment lipidiques; une lotion biphase ou multiphase; un spray; une poudre libre, compacte ou coulée; une pâte anhydre.

**14.** Composition selon l'une des revendications 11 à 13, se présentant sous la forme d'un produit de soin et/ou de maquillage de la peau tel qu'un rouge à lèvres, un fond de teint, un fard à joues ou à paupières, une poudre libre ou compacte, une crème teintée, un eye-liner, un mascara, un vernis à ongles; un produit solaire ou autobronzant, un produit capillaire.

## Claims

**1.** Process for preparing a photochromic compound having a parameter $\Delta(\Delta E)$ greater than or equal to 7, in which an initially photochromic compound of the metal oxide, hydrated metal oxide or metal oxide and/or hydrate complex type is heat-treated at a temperature of 400-1000°C in the presence of a metallic component selected from the oxides and/or hydroxides of lithium, sodium and/or potassium.

**2.** Process for improving the parameter $\Delta(\Delta E)$ of an initially photochromic compound of the metal oxide, hydrated metal oxide or metal oxide and/or hydrate complex type, consisting in heat-treating the said compound at a temperature of 400-1000°C in the presence of a metallic component selected from the oxides and/or hydroxides of lithium, sodium and/or potassium.

**3.** Process according to one of the preceding claims, in which the photochromic compound is selected from the oxides or hydrated oxides of titanium, niobium, silicon, aluminium, zinc, hafnium, thorium, tin, thallium, zirconium, beryllium, cobalt, calcium and magnesium.

**4.** Process according to one of the preceding claims, in which the photochromic compound is a titanium dioxide rendered photochromic using a metal selected from iron, chromium, copper, nickel, manganese, cobalt, molybdenum, as such or in the form of a salt such as a sulphate, a chlorate, a nitrate or an acetate.

**5.** Process according to one of the preceding claims, in which the metallic component is selected from the oxides or hydroxides of lithium.

**6.** Process according to one of the preceding claims, in which the metallic component is present in a ratio of 0.01 - 30% by weight of metal ions, preferably 0.02 - 20%, with respect to the weight of photochromic compound to be treated.

**7.** Process according to one of the preceding claims, in which the heat treatment is carried out at a temperature of 600 - 900°C.

**8.** Process according to one of the preceding claims, in which the heat treatment is carried out for from 10 minutes to 6 hours, preferably 2 - 5 hours.

**9.** Photochromic compound of the metal oxide, hydrated metal oxide or metal oxide and/or hydrated complex type, which can be obtained using the process according to one of Claims 1 to 8.

**10.** Compound according to Claim 9, having a parameter $\Delta(\Delta E)$ greater than or equal to 10, preferably greater than or equal to 12.

**11.** Cosmetic composition comprising the compound according to one of Claims 9 and 10.

**12.** Composition according to Claim 11, comprising 0.01 to 30% by weight, preferably 1 to 15% by weight, of the compound according to one of Claims 9 and 10.

**13.** Composition according to one of Claims 11 and 12, which is in the form of a suspension, a dispersion or a solution in solvent or aqueous-alcoholic medium, optionally thickened or gelled; an oil-in-water, water-in-oil or multiple emulsion; a gel or a foam; an emulsified gel; a dispersion of vesicles, in particular lipid vesicles; a two-phase or multi-phase lotion; a spray; a free, compact or loose powder; an anhydrous paste.

**14.** Composition according to one of Claims 11 to 13, which is in the form of a care and/or make-up product for the skin, such as a lipstick, a foundation, a blusher or eye-shadow, a free or compact powder, a tinted cream, an eyeliner, a mascara, a nail varnish; a suncare or self-tanning product, a haircare product.

**Patentansprüche**

**1.** Verfahren zur Herstellung einer photochromen Verbindung mit einem Parameter $\Delta(\Delta E)$ größer oder gleich 7, nach dem eine ursprünglich photochrome Verbindung vom Typ eines Metalloxids, eines Metalloxidhydrats oder eines Metalloxid- und/oder -hydratkomplexes in Gegenwart einer metallischen Komponente, die unter den Oxiden und/ oder Hydroxiden von Lithium, Natrium und/oder Kalium ausgewählt ist, bei einer Temperatur von 400 bis 1000 °C thermisch behandelt wird.

**2.** Verfahren zur Verbesserung des Parameters $\Delta(\Delta E)$ einer ursprünglich photochromen Verbindung vom Typ eines Metalloxids, eines Metalloxidhydrats oder eines Metalloxid- und/oder -hydratkomplexes, das darin besteht, diese Verbindung in Gegenwart einer metallischen Komponente, die unter den Oxiden und/oder Hydroxiden von Lithium, Natrium und/oder Kalium ausgewählt ist, bei einer Temperatur von 400 bis 1000 °C thermisch zu behandeln.

**3.** Verfahren nach einem der vorhergehenden Ansprüche, wobei die photochrome Verbindung ausgewählt ist unter den Oxiden oder Oxidhydraten von Titan, Niob, Silicium, Aluminium, Zink, Hafnium, Thorium, Zinn, Thallium, Zirkonium, Beryllium, Cobalt, Calcium und Magnesium.

4. Verfahren nach einem der vorhergehenden Ansprüche, wobei die photochrome Verbindung Titandioxid ist, das mit einem Metall zu einer photochromen Verbindung gemacht wurde, welches ausgewählt ist unter Eisen, Chrom, Kupfer, Nickel, Mangan, Cobalt und Molybdän, wobei dieses Metall als solches oder in Form eines Salzes, wie als Sulfat, Chlorat, Nitrat oder Acetat, vorliegt.

5. Verfahren nach einem der vorhergehenden Ansprüche, wobei die metallische Komponente unter Lithiumoxiden oder Lithiumhydroxiden ausgewählt ist.

6. Verfahren nach einem der vorhergehenden Ansprüche, wobei die metallische Komponente in einem Anteil von 0,01 bis 30 Gew.-% ausgedrückt als Metallion, vorzugsweise 0,02 bis 20 %, bezogen auf das Gewicht der zu behandelnden photochromen Verbindung, vorliegt.

7. Verfahren nach einem der vorhergehenden Ansprüche, wobei die thermische Behandlung bei einer Temperatur von 600 bis 900 °C durchgeführt wird.

8. Verfahren nach einem der vorhergehenden Ansprüche, wobei die thermische Behandlung während einer Zeitspanne von 10 Minuten bis 6 Stunden, vorzugsweise von 2 bis 5 Stunden, durchgeführt wird.

9. Photochrome Verbindung vom Typ eines Metalloxids, eines Metalloxidhydrats oder eines Metalloxid- und/oder -hydratkomplexes, die mit dem Verfahren nach einem der Ansprüche 1 bis 8 hergestellt werden kann.

10. Verbindung nach Anspruch 9, die einen Parameter $\Delta(\Delta E)$ von mindestens 10, vorzugsweise von mindestens 12, aufweist.

11. Kosmetische Zusammensetzung, welche die Verbindung nach einem der Ansprüche 9 bis 10 enthält.

12. Zusammensetzung nach Anspruch 11, die 0,01 bis 30 Gew.-%, vorzugsweise 1 bis 15 Gew.-%, der Verbindung nach einem der Ansprüche 9 bis 10 enthält.

13. Zusammensetzung nach einem der Ansprüche 11 bis 12, die in Form einer Suspension, einer Dispersion, einer Lösung in einem Lösungsmittelmedium oder einem wässerigalkoholischen Medium, die gegebenenfalls verdickt oder sogar geliert ist; einer Öl-in-Wasser-Emulsion, einer Wasser-in-Öl-Emulsion oder einer multiplen Emulsion; eines Gels oder eines Schaums; eines emulgierten Gels, einer Vesikeldispersion, insbesondere einer Lipidvesikeldispersion; einer Zweiphasenlotion oder einer Mehrphasenlotion, eines Sprays; eines losen, gepreßten oder gegossenen Pulvers oder einer wasserfreien Paste vorliegt.

14. Zusammensetzung nach einem der Ansprüche 11 bis 13, die in Form eines Pflegeproduktes und/oder Schminkproduktes für die Haut, wie als Lippenstift, Make-up, Wangenrouge, Lidschatten, loses oder gepreßtes Pulver, getönte Creme, Eyeliner, Mascara, Nagellack, Sonnenschutzprodukt oder Selbstbräunungsprodukt oder als Produkt zur Haarbehandlung vorliegt.